# EUROPÄISCHE PATENTSCHRIFT

(11) **EP 2 785 684 B1**
(45) Veröffentlichungstag und Bekanntmachung des Hinweises auf die Patenterteilung: **18.11.2015**
(21) Anmeldenummer: 12794938.6
(22) Anmeldetag: 28.11.2012
(51) Int. Cl.: C07C 263/10

(54) **VERFAHREN ZUR HERSTELLUNG VON ISOCYANATEN DURCH PHOSGENIERUNG DER ENTSPRECHENDEN AMINE IN DER GASPHASE**
METHOD FOR THE PRODUCTION OF ISOCYANATES BY MEANS OF PHOSGENATION OF THE RESPECTIVE AMINES IN THE GASEOUS PHASE
PROCÉDÉ DE PRODUCTION D'ISOCYANATES PAR PHOSGÉNATION DES AMINES CORRESPONDANTES EN PHASE GAZEUSE

(30) Priorität: 29.11.2011 EP 11191097
(43) Veröffentlichungstag der Anmeldung: 08.10.2014
(73) Patentinhaber: BASF SE, 67056 Ludwigshafen (DE)
(72) Erfinder: MATTKE, Torsten, 67251 Freinsheim (DE); KNÖSCHE, Carsten, 67150 Niederkirchen (DE); LEHR, Vanessa, Simone, 68199 Mannheim (DE)
(86) Internationale Anmeldenummer: PCT/EP2012/073809
(87) Internationale Veröffentlichungsnummer: WO 2013/079517

(56) Entgegenhaltungen:
- EP-A1- 1 526 129
- WO-A1-2009/027234

## Beschreibung

Die Erfindung betrifft ein Verfahren zur Herstellung von Isocyanaten durch Phosgenierung der entsprechenden Amine.

Die Phosgenierung ist das am weitaus häufigsten industriell eingesetzte Verfahren zur Synthese von Isocyanaten. Isocyanate, insbesondere Diisocyanate, werden überwiegend als Ausgangsstoffe in der Polyurethanindustrie eingesetzt.

Nach den bekannten Verfahren wird die Phosgenierung in der Regel in der Flüssigphase durchgeführt.

Neuere Verfahren (WO-A-2009/027234) arbeiten auch bereits in der Gasphase, da die Gasphasenphosgenierung gegenüber der Flüssigphasenphosgenierung eine Reihe von Vorteilen aufweist:

In der Flüssigphasenphosgenierung fallen durch Reaktion der eingesetzten Amine mit dem in der Phosgenierung abgespaltenen Chlorwasserstoff Amin-Hydrochloride als Feststoffe aus. Die Amin-Hydrochloride reagieren zwar auch mit Phosgen weiter zum Zielprodukt Isocyanat, da es sich hierbei jedoch um eine langsame Feststoffreaktion handelt finden auch verstärkt Nebenreaktionen statt, insbesondere die Reaktion der Amin-Hydrochloride mit dem Zielprodukt Isocyanat zu Harnstoffen.

Demgegenüber liegt bei der Gasphasenphosgenierung das Gleichgewicht Amin zu Amin-Hydrochlorid stark auf der Amin-Seite, so dass wesentlich weniger Nebenprodukte gebildet werden und die Ausbeuten entsprechend höher sind. Darüber hinaus ist bei der Gasphasenphosgenierung auch der Hold-up an toxischem Phosgen im Vergleich zur Flüssigphasenphosgenierung niedriger.

Die Gasphasenphosgenierung ist jedoch gegenüber der Flüssigphasenphosgenierung verfahrenstechnisch wesentlich anspruchsvoller: insbesondere ist die Zersetzungsgefahr für das Amin sowie für das gebildete Isocyanat sehr groß, so dass das Amin sehr schnell verdampft werden muss. Hierzu sind sehr große spezifische Oberflächen erforderlich. Darüber hinaus müssen sehr kurze, definierte Verweilzeiten im Hochtemperaturbereich während der Reaktion gewährleistet werden, und das Produktgemisch muss nach der Reaktion sehr schnell abgekühlt werden.

Es war Aufgabe der Erfindung, ein Verfahren zur Herstellung von Isocyanaten durch Phosgenierung der entsprechenden Amine in der Gasphase zur Verfügung zu stellen, das die obigen Herausforderungen bewältigt.

Die Lösung besteht in einem Verfahren zur Herstellung von Isocyanaten durch Phosgenierung der entsprechenden Amine in einem Wirbelschichtreaktor, das dadurch gekennzeichnet ist, dass ein Gasstrom, enthaltend das Phosgen, als Wirbelgas genutzt wird und einen inerten Feststoff in der Schwebe hält, und dass ein flüssiger Strom, enthaltend das Amin, in die Wirbelschicht dosiert wird, wobei das Amin teilweise oder vollständig verdampft und mit dem Phosgen unter Erhalt eines Reaktionsgasgemisches, enthaltend das entsprechende Isocyanat, reagiert, das aus dem Wirbelschichtreaktor abgezogen wird.

Indem erfindungsgemäß das Edukt Amin in flüssigem Zustand in die durch die Reaktionswärme der Phosgenierungsreaktion aufgeheizte Wirbelschicht eingesprüht wird, wird das Amin sehr schnell verdampft und überhitzt, so dass die Gefahr von Ablagerungen auf dem inerten Feststoff, der die Wirbelschicht ausbildet, gering ist. Der inerte Feststoff kann ausgeschleust und mögliche Ablagerungen abgebrannt werden. Insgesamt handelt es sich um ein sehr robustes System, für das nicht die Gefahr besteht, dass es sich durch Ablagerungen zusetzt.

Indem das Verfahren die Reaktionswärme der Phosgenierungsreaktion selbst nutzt, wird die sehr teure Hochtemperaturenergie, die in bislang bekannten Verfahren durch Quenchen des Reaktionsgemisches der Gasphasenphosgenierung vernichtet wurde, am Ort ihrer Erstehung genutzt, um die Edukte auf die Betriebstemperatur der Gasphasenphosgenierung zu bringen.

Darüber hinaus werden auch die entsprechenden Wärmetauscher eingespart oder können kleiner dimensioniert werden.

Die bei der Gasphasenphosgenierung freiwerdende Reaktionswärme wird durch die Zirkulationen in der Wirbelschicht in Bereiche zur Aufwärmung, Verdampfung bzw. Überhitzung der Eduktströme transportiert.

Ein weiterer Vorteil der erfindungsgemäßen Verfahrensführung in einer Wirbelschicht besteht darin, dass eine adiabate Temperaturerhöhung des Reaktionsgemisches und damit hohe Austrittstemperaturen desselben vermieden werden können.

Bevorzugt ist der Wirbelschichtreaktor ein rotationssymmetrischer Apparat mit vertikaler Längsachse. Weiter bevorzugt ist der Wirbelschichtreaktor, ein überwiegend zylindrischer Apparat.

Bevorzugt wird der phosgenhaltige Gasstrom, der sowohl das Edukt-Phosgen enthält als auch als Wirbelgas im Wirbelschichtreaktor eingesetzt wird, vor der Zuführung desselben zum Wirbelschichtreaktor, auf eine Temperatur erhitzt, die um mindestens 5 °C größer ist als die Verdampfungstemperatur des Amins unter den Betriebsbedingungen im Wirbelschichtreaktor.

Der phosgenhaltige Gasstrom enthält bevorzugt 50 bis 100 Gew.-% Phosgen bezogen auf das Gesamtgewicht des phosgenhaltigen Gasstromes, bevorzugt 75 bis 100 Gew.-% Phosgen bezogen auf das Gesamtgewicht des phosgenhaltigen Gasstromes, weiter bevorzugt 90 bis 99,9 Gew.-% Phosgen, bezogen auf das Gesamtgewicht des phosgenhaltigen Gasstromes.

Der aminhaltige flüssige Strom, der in die Wirbelschicht dosiert wird, bevorzugt unmittelbar vor der Dosierung desselben in die Wirbelschicht, wird auf eine Temperatur vorerhitzt, die um mindestens 1 °C, bevorzugt mindestens 5 °C, unter der Siedetemperatur des Amins, unter den Betriebsbedingungen im Wirbelschichtreaktor liegt.

Bevorzugt enthält der aminhaltige flüssige Strom 50 bis 100 Gew.-% Amin, bezogen auf das Gesamtgewicht des aminhaltigen flüssigen Stromes, weiter bevorzugt 70 bis 100 Gew.-% Amin, bezogen auf das Gesamtgewicht des aminhaltigen flüssigen Stromes, insbesondere 90 bis 99,99 Gew.-% Amin, bezogen auf das Gesamtgewicht des aminhaltigen flüssigen Stromes.

Amine, die bei dem erfindungsgemäßen Verfahren zur Reaktion zu den korrespondierenden Isocyanaten eingesetzt werden, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höchstens 1 Mol-% und ganz besonders bevorzugt zu höchstens 0,5 Mol-% zersetzen. Besonders geeignet sind hier Amine, insbesondere Diamine, auf Basis von (cyclo)aliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-amino-methylcyclohexan (IP-DA) und 4,4-Diaminodicyclohexylmethan. Bevorzugt verwendet wird 1,6-Diaminohexan (Hexamethylendiamin, HDA).

Für das erfindungsgemäße Verfahren können ebenfalls aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphthyldiamin (NDA) und 2,4'- oder 4,4'-Methylen(diphenyldiamin) (MDA) oder Isomerengemische davon. Bevorzugt sind unter diesen die Diamine, besonders bevorzugt sind 2,4- und/oder 2,6-TDA oder 2,4'-und/oder 4,4'-MDA.

Zur Herstellung von Monoisocyanaten können ebenfalls aliphatische, cycloaliphatische oder aromatische Amine, üblicherweise Monoamine, eingesetzt werden. Als aromatisches Monoamin ist insbesondere Anilin bevorzugt.

Weiter können auch Triamine oder höhere Amine eingesetzt werden.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Carbamoyl- und Aminhydrochloride), Endprodukte (Diisocyanate) sowie gegebenenfalls zudosierte inerte Verbindungen, unter den Reaktionsbedingungen in der Gasphase verbleiben. Sollten diese oder andere Komponenten sich aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für Aminhydrochloride, die sich aus freien Aminogruppen und Chlorwasserstoff bilden, da diese leicht ausfallen und nur schwer wieder verdampfbar sind.

Um die Bildung von Nebenprodukten zu vermeiden, ist es bevorzugt, Phosgen im Überschuss zuzuführen. Um nur den für die Reaktion notwendigen Anteil an Aminen zuzuführen, ist es möglich, das Amin mit einem Inertgas zu mischen. Durch den Anteil an Inertgas im Amin lässt sich die Menge des zugeführten Amins bei vorgegebener Geometrie der Zufuhröffnungen für das Amin und das Phosgen einstellen. Inertmedien, die zugegeben werden können, sind solche, die im Reaktionsraum gasförmig vorliegen und nicht mit den im Reaktionsverlauf auftretenden Verbindungen reagieren. Als Inertmedium können zum Beispiel Stickstoff, Edelgase wie Helium oder Argon, Aromaten wie Chlorbenzol, o-Dichlorbenzol, Trichlorbenzol, Toluol, Xylol, Chlornaphtalin, Decahydronaphtalin, Kohlenstoffdioxid oder Kohlenstoffmonoxid eingesetzt werden. Bevorzugt werden Stickstoff und/oder Chlorbenzol als Inertmedium verwendet.

Alternativ ist es jedoch auch möglich, zum Beispiel um einen zu großen Überschuss an Phosgen zu vermeiden, ein Inertmedium, bevorzugt Stickstoff, dem Phosgen zuzumischen.

Im Allgemeinen wird das Inertmedium in einer Menge zugesetzt, dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5 beträgt.

Das Verfahren wird in einer vorteilhaften Ausführungsform in der Weise durchgeführt, dass aus dem Wirbelschichtreaktor im oberen Bereich desselben ein Gemisch ausströmt, das das Reaktionsgasgemisch sowie den inerten Feststoff enthält, und das einer Auftrennung zugeführt wird.

Bevorzugt wird die Auftrennung in das Reaktionsgasgemisch einerseits und den inerten Feststoff andererseits in einem oder mehreren Apparaten, ausgewählt aus der Gruppe Zyklon und Filter, durchgeführt.

Im abgetrennten Reaktionsgasgemisch sind bevorzugt 50 bis 100 % , weiter bevorzugt 75 bis 100 %, besonders bevorzugt 90 bis 100 % der eingesetzten Amingruppen umgesetzt.

Vorteilhaft wird das Reaktionsgasgemisch einem anschließenden Verweilzeitreaktor zur vollständigen Umsetzung der Amingruppen zugeführt.

Weiter vorteilhaft kann das Reaktionsgasgemisch durch direkten oder indirekten Wärmeeintrag in einer oder mehreren Stufen heruntergekühlt und dabei gegebenenfalls eine Flüssigphase auskondensiert werden.

Der abgetrennte Feststoff kann vorteilhaft teilweise oder vollständig in den unteren Teil der Wirbelschicht zurückgeführt werden.

Bevorzugt wird der Feststoff teilweise oder vollständig aufgearbeitet, insbesondere in einer zweiten Wirbelschicht, und der aufgearbeitete Feststoff wird bevorzugt teilweise oder vollständig in den Wirbelschichtreaktor recycliert. Besonders bevorzugt erfolgt die Aufarbeitung des Feststoffs im Wesentlichen durch Abbrand kohlenstoffhaltiger Ablagerungen auf dem Feststoff.

Bei dem inerten Feststoff handelt es sich bevorzugt um ein gegenüber allen Reaktionspartnern und -produkten inertes Material, bevorzugt um ein anorganisches Material, besonders bevorzugt um SiO₂ oder Al₂O₃, insbesondere α-Al₂O₃, handelt.

Die mittlere Partikelgröße des inerten Feststoffs liegt insbesondere im Bereich von 20 µm bis 2000 µm, bevorzugt im Bereich von 20 µm bis 500 µm, und besonders bevorzugt im Bereich von 50 µm bis 150 µm.

Der flüssige Strom enthaltend das Amin wird insbesondere mittels einer oder mehrerer Düsen, die als Einstoff- oder Zweistoffdüsen, bevorzugt als Zweistoffdüsen, ausgebildet sind, in die Wirbelschicht eindosiert, wobei das Zerstäubungsgas der Zweistoffdüsen vorzugsweise ein Phosgen enthaltendes Gas, weiter bevorzugt überhitztes Amin, ein Inertgas oder Mischungen hiervon, ist.

Die Zweistoffdüsen im Falle eines Phosgen enthaltenden Gases als Zerstäubungsgas sind bevorzugt als außen mischend ausgeführt.

Beim Einsatz von zwei oder mehreren Düsen für die Eindosierung des flüssigen Stromes, enthaltend das Amin, sind dieselben vorteilhaft über den Querschnitt gleichmäßig verteilt anzubringen.

Die Erfindung wird im Folgenden anhand von Zeichnungen sowie von Ausführungsbeispielen näher erläutert.

Es zeigen im Einzelnen:
- Figur 1: ein Fließschema für eine bevorzugte Verfahrensführung mit Wärmeintegration,
- Figur 2: eine schematische Darstellung einer bevorzugten Ausführungsform des Reaktors mit zirkulierender Wirbelschicht, und
- Figur 3: eine schematische Darstellung des Temperaturprofils im Riser für ein Ausführungsbeispiel in einem zirkulierenden Wirbelschicht-Set-up entsprechend Figur 2.

In den Figuren bezeichnen gleiche Bezugszeichen gleiche oder entsprechende Merkmale.

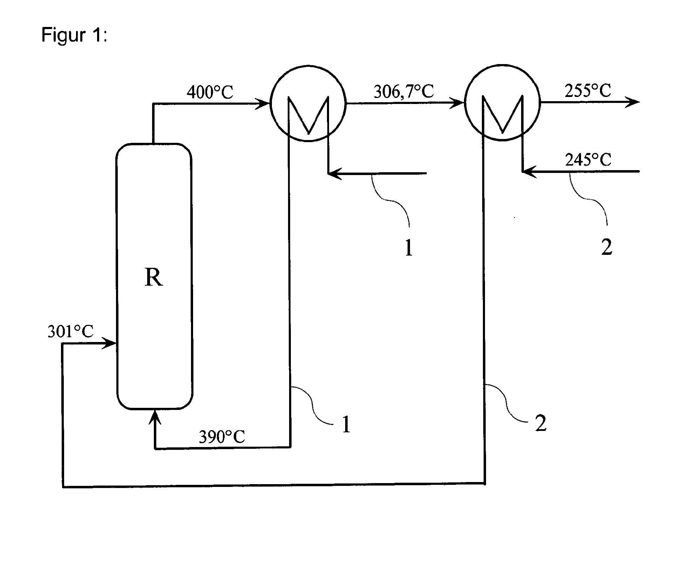

Figur 1 zeigt ein Fließschema für eine bevorzugte Verfahrensführung mit Wärmeintegration. In einen schematisch dargestellten Wirbelschichtreaktor R wird ein Phosgen enthaltener Gasstrom 1, der auf 390 °C vorgeheizt ist, eingeleitet. Beispielhaft wird Toluylendiamin enthaltender Strom 2 mit 40 bar Dampf auf 245 °C aufgeheizt und anschließend mit dem Reaktionsgemisch der Phosgenierung auf 301 °C aufgeheizt, wobei er noch in flüssigem Aggregatzustand verbleibt. Anschließend wird der Strom 2 in den Wirbelschichtreaktor eingedüst. Die Verdampfung des Toluylendiamins und die Reaktion desselben mit dem Phosgen erfolgen bei einer Temperatur von etwa 400 °C im Wirbelschichtreaktor. Dabei entspricht die freiwerdende Reaktionswärme der zur Verdampfung und Aufheizung der Edukte benötigten Wärme.

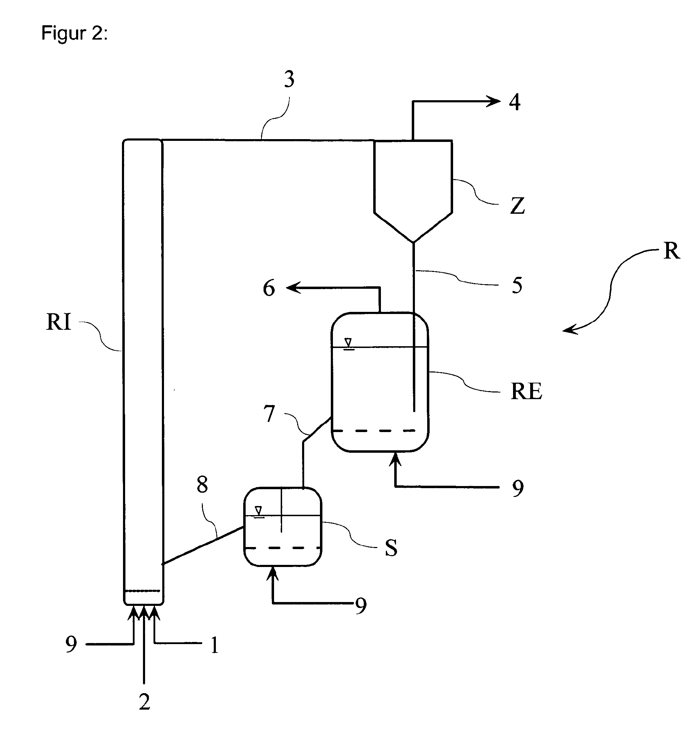

Figur 2 zeigt einen bevorzugten Wirbelschichtreaktor R, der als zirkulierendes Wirbelschicht-Set up ausgebildet ist, umfassend einen Riser RI, einen Zyklon Z, eine blasenbindende Wirbelschicht als Regenerator RE und einen Siphon S.

In den Riser RI wird am unteren Ende desselben ein Phosgen enthaltender Gasstrom 1 und ein Toluylendiamin enthaltender Strom 2 sowie ein inerter Feststoffstrom 8 zugeführt. Das Feststoffpartikel-Reaktionsgasgemisch, Strom 3, wird vom oberen Ende des Risers RI in den nachgeschalteten Zyklon Z eingeleitet. Aus dem Zyklon Z wird über den Oberlauf ein Produktstrom 4 abgezogen und über den Unterlauf der den zirkulierenden Feststoff umfassende Strom 5. Dieser wird über ein Fallrohr in eine blasenbildende Wirbelschicht, die als Regenerator RE fungiert, eingeleitet. Aus dem oberen Bereich des Regenerators RE wird ein Abgasstrom, Strom 6, ausgeschleust.

Aus dem Regenerator RE wird im unteren Bereich desselben ein den zirkulierenden inerten Feststoff enthaltender Strom 7 über einen Siphon S als Strom 8 in den unteren Bereich des Risers RI recycliert.

Das Fallrohr (Standpipe) und der Siphon S werden mit Inertgas, Strom 9, fluidisiert und somit als Stripper betrieben. Die als Regenerator RE eingesetzte blasenbildende Wirbelschicht kann ebenfalls mit Inertgas, Strom 9, oder auch mit Luft Strom 10 anstelle von Inertgas, betrieben werden. Im Falle eines Betriebes mit Luft werden kohlenstoffhaltige Ablagerungen auf den Feststoffpartikeln abgebrannt. Der Riser RI kann zur Inertisierung und zur Fluidisationsstützung ebenfalls mit Inertgas beaufschlagt werden.

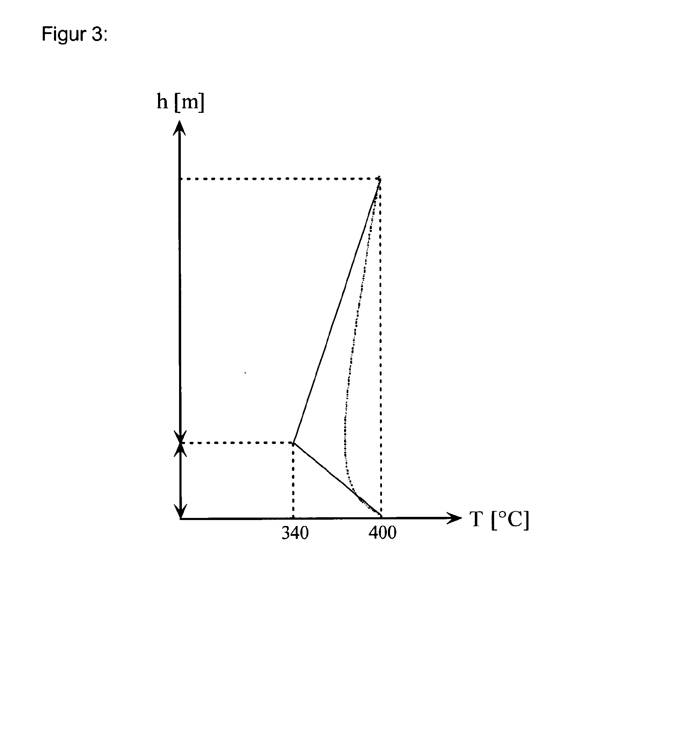

Figur 3 zeigt ein charakteristisches Temperaturprofil im Riser RI, wobei auf der Abszisse die Temperatur T in °C und auf der Ordinate die Höhe h des Risers RI in Metern angegeben ist.

### Ausführungsbeispiele

Die Ausführungsbeispiele beziehen sich auf einen Reaktor entsprechend der schematischen Darstellung in Figur 2.

Der Anlagendruck im Riser RI beträgt 3 bar absolut, die Reaktionstemperatur 400 °C.

Die Eduktströme sind auf 1,8 kg/h Toluylendiamin (Strom 2) und 9 kg/h Phosgen (Strom 1) festgelegt. Das Phosgen wird vollständig in der Gasphase zugeführt, das Toluylendiamin vollständig in der flüssigen Phase.

Phosgen (Strom 1) und Toluylendiamin (Strom 2) werden mittels zweier Einstoffdüsen in den unteren Bereich des Risers RI gebracht. Alternativ können Phosgen (Strom 1) und Toluylendiamin (Strom 2) auch mittels einer Zweistoffdüse in den unteren Bereich des Risers RI gebracht werden. Zur Fluidisierung dient Stickstoff (Strom 9). Alternativ kann zur Fluidisierung auch Luft eingesetzt werden.

Die eingesetzten inerten Feststoffpartikel haben einen Sauter-Durchmesser von 65 µm und eine Scheindichte, d.h. die mittlere Dichte eines nicht homogenen, porösen Partikels bezogen auf seine äußere Umhüllende, von 1900 kg/m³. Die Zirkulationsrate ergibt sich aus Kaltversuchen zu ca. 50 kg/m²/s.

Unter den obigen Bedingungen beträgt die Gasgeschwindigkeit im Riser RI ca. 2 m/s und die Gasverweilzeit entsprechend 2 s.

Das charakteristische Temperaturprofil im Riser RI für das obige Ausführungsbeispiel ist in Figur 3 wiedergegeben.

Der Siedepunkt von Toluylendiamin liegt bei 3 bar absolut bei ca. 337 °C. Unter der extremen Annahme, dass die Verdampfung des Toluylendiamins vor der Reaktion erfolgt und 295 W zum Aufheizen und Verdampfen von Phosgen und Toluylendiamid sowie ein cₚ des inerten Feststoffes von 1,5 kJ/kgK benötigt werden, ergibt sich eine lokale Temperaturabnahme auf mindestens 385 °C. Somit ist von einem kontinuierlichen Temperaturgefälle von 40 bis 70 °C bei der Verdampfung auszugehen. Der Betrieb im Riser ist im unteren Bereich desselben verdampfungsdominiert und im oberen Bereich desselben reaktionsdominiert.

Die Energiebilanz für verschiedene Temperaturen ist in der nachfolgenden Tabelle wiedergegeben.

| Toluylendiamin | | Phosgen | | Toluylendiamin Aufheizen und Verdampfen auf Zieltemperatur | Phosgen Aufheizen auf Zieltemperatur | Heizleistung | |
|---|---|---|---|---|---|---|---|
| Tₑᵢₙ [°C] | Tₐᵤₛ [°C] | Tₑᵢₙ [°C ] | Tₐᵤₛ [°C ] | Heizleistung [W] | Heizleistung [W] | Gesamt [W] | Reaktionswärme [W] |
| 332 | 400 | 400 | 400 | 295 | 0 | 295 | 295 |
| 336 | 350 | 314 | 350 | 233 | 65 | 297 | 295 |
| 313 | 338 | 313 | 338 | 252 | 45 | 296 | 295 |

Überraschenderweise geht die Energiebilanz für den Fall einer bevorzugten Reaktionstemperatur von 400 °C und einer Flüssigeindüsung des Toluylendiamins leicht unterhalb der Verdampfungstemperatur (ca. 5 °C) und Vorheizen der Gasströme auf Reaktortemperatur exakt auf.

## Patentansprüche

1. Verfahren zur Herstellung von Isocyanaten durch Phosgenierung der entsprechenden Amine in einem Wirbelschichtreaktor (R), **dadurch gekennzeichnet, dass**
- ein Gasstrom (1), enthaltend das Phosgen, als Wirbelgas eingesetzt wird und einen inerten Feststoff in der Schwebe hält, und dass
- ein flüssiger Strom (2), enthaltend das Amin, in die Wirbelschicht dosiert wird,
- wobei das Amin teilweise oder vollständig verdampft und mit dem Phosgen unter Erhalt eines Reaktionsgasgemisches, enthaltend das entsprechende Isocyanat, reagiert, das aus dem Wirbelschichtreaktor (R) abgezogen wird.

2. Verfahren nach Anspruch 1, **dadurch gekennzeichnet, dass** der Wirbelschichtreaktor (R) ein rotationssymmetrischer Apparat mit vertikaler Längsachse ist.

3. Verfahren nach Anspruch 2, **dadurch gekennzeichnet, dass** der Wirbelschichtreaktor (R) ein überwiegend zylindrischer Apparat ist.

4. Verfahren nach einem der Ansprüche 1 bis 3, **dadurch gekennzeichnet, dass** der phosgenhaltige Gasstrom (1), enthaltend 50 bis 100 Gew.-% Phosgen, bezogen auf das Gesamtgewicht des phosgenhaltigen Gasstroms, bevorzugt 75 bis 100 Gew.-% Phosgen, bezogen auf das Gesamtgewicht des phosgenhaltigen Gasstroms, weiter bevorzugt 90 bis 99,9 Gew.-% Phosgen, bezogen auf das Gesamtgewicht des phosgenhaltigen Gasstroms, vor der Zuführung desselben zum Wirbelschichtreaktor auf eine Temperatur erhitzt wird, die um mindestens 5 °C größer ist als die Verdampfungstemperatur des Amins unter den Betriebsbedingungen (Druck) im Wirbelschichtreaktor (R).

5. Verfahren nach einem der Ansprüche 1 bis 4, **dadurch gekennzeichnet, dass** der aminhaltige flüssige Strom (2), enthaltend 50 bis 100 Gew.-% Amin, bezogen auf das Gesamtgewicht des aminhaltigen flüssigen Stroms, bevorzugt 75 bis 100 Gew.-% Amin, bezogen auf das Gesamtgewicht des aminhaltigen flüssigen Stroms, weiter bevorzugt 90 bis 99,99 Gew.-% Amin, bezogen auf das Gesamtgewicht des aminhaltigen flüssigen Stroms, unmittelbar vor der Dosierung in die Wirbelschicht auf eine Temperatur vorerhitzt wird, die um mindestens 1 °C, bevorzugt mindestens 5 °C, unter der Siedetemperatur des Amins unter den Betriebsbedingungen im Wirbelschichtreaktor liegt.

6. Verfahren nach einem der Ansprüche 1 bis 5, **dadurch gekennzeichnet, dass** aus dem Wirbelschichtreaktor (R) im oberen Bereich desselben ein Gemisch ausströmt, das das Reaktionsgasgemisch sowie den inerten Feststoff enthält, und das einer Auftrennung zugeführt wird.

7. Verfahren nach Anspruch 6, **dadurch gekennzeichnet, dass** die Auftrennung in das Reaktionsgasgemisch einerseits und den inerten Feststoff andererseits in einem oder mehreren Apparaten, ausgewählt aus der Gruppe Zyklon und Filter, durchgeführt wird.

8. Verfahren nach einem der Ansprüche 1 bis 7, **dadurch gekennzeichnet, dass** im abgetrennten Reaktionsgasgemisch 50 bis 100 %, bevorzugt 75 bis 100 %, besonders bevorzugt 90 bis 100% der eingesetzten Amingruppen umgesetzt sind.

9. Verfahren nach einem der Ansprüche 1 bis 8, **dadurch gekennzeichnet, dass** das Reaktionsgasgemisch einem anschließenden Verweilzeitreaktor zur vollständigen Umsetzung der Amingruppen zugeführt wird.

10. Verfahren nach einem der Ansprüche 1 bis 9, **dadurch gekennzeichnet, dass** das Reaktionsgasgemisch durch direkte oder indirekte Wärmeübertragung in einer oder mehreren Stufen heruntergekühlt und dabei gegebenenfalls eine Flüssigphase auskondensiert wird.

11. Verfahren nach einem der Ansprüche 1 bis 10, **dadurch gekennzeichnet, dass** der Feststoff teilweise oder vollständig in den unteren Teil der Wirbelschicht zurückgeführt wird.

12. Verfahren nach einem der Ansprüche 1 bis 11, **dadurch gekennzeichnet, dass** der Feststoff teilweise oder vollständig aufgearbeitet wird, bevorzugt in einer zweiten Wirbelschicht, und dass der aufgearbeitete Feststoff teilweise oder vollständig in den Wirbelschichtreaktor (R) recycliert wird, besonders bevorzugt, dass die Aufarbeitung des Feststoffs im Wesentlichen durch Abbrand kohlenstoffhaltiger Ablagerungen auf dem Feststoff erfolgt.

13. Verfahren nach einem der Ansprüche 1 bis 12, **dadurch gekennzeichnet, dass** es sich bei dem inerten Feststoff um ein gegenüber allen Reaktionspartnern und -produkten inertes Material, bevorzugt um ein anorganisches Material, besonders bevorzugt um SiO₂ oder Al₂O₃, insbesondere α-Al₂O₃, handelt.

14. Verfahren nach einem der Ansprüche 1 bis 13, **dadurch gekennzeichnet, dass** die mittlere Partikelgröße des inerten Feststoffs im Bereich von 20 µm bis 2000 µm, bevorzugt im Bereich von 20 µm bis 500 µm, und besonders bevorzugt im Bereich von 50 µm bis 150 µm, liegt.

15. Verfahren nach einem der Ansprüche 1 bis 14, **dadurch gekennzeichnet, dass** das sich bei dem eingesetzten Amin um ein Diamin, bevorzugt um Hexamethylendiamin, Isomere und Isomerengemische des Toluylendiamins oder Methylendiphenyldiamins, besonders bevorzugt um ein Isomerengemisch des Toluylendiamins, handelt.

16. Verfahren nach einem der Ansprüche 1 bis 15, **dadurch gekennzeichnet, dass** der aminhaltige und/oder der phosgenhaltige Eduktstrom mit einer inerten Komponente, bevorzugt Stickstoff, verdünnt ist.

17. Verfahren nach einem der Ansprüche 1 bis 16, **dadurch gekennzeichnet, dass** der flüssige Strom enthaltend das Amin mittels einer oder mehrerer Düsen, die als Einstoff- oder Zweistoffdüsen, bevorzugt als Zweistoffdüsen, ausgebildet sind, in die Wirbelschicht eindosiert wird, wobei das Zerstäubungsgas der Zweistoffdüsen vorzugsweise ein Phosgen enthaltendes Gas ist.

18. Verfahren nach Anspruch 17, **dadurch gekennzeichnet, dass** die Zweistoffdüsen im Falle eines Phosgen enthaltenden Gases als Zerstäubungsgas bevorzugt als außen mischend ausgeführt sind.

## Claims

1. A process for preparing isocyanates by phosgenation of the corresponding amines in a fluidized-bed reactor (R), wherein
- a gas stream (1) comprising the phosgene is used as fluidizing gas and keeps an inert solid in suspension and
- a liquid stream (2) comprising the amine is fed into the fluidized bed,
- with the amine vaporizing partially or completely and reacting with the phosgene to give a reaction gas mixture which comprises the corresponding isocyanate and is taken off from the fluidized-bed reactor (R).

2. The process according to claim 1, wherein the fluidized-bed reactor (R) is a rotationally symmetrical apparatus having a vertical longitudinal axis.

3. The process according to claim 2, wherein the fluidized-bed reactor (R) is a predominantly cylindrical apparatus.

4. The process according to any of claims 1 to 3, wherein the phosgene-comprising gas stream (1) comprising from 50 to 100% by weight of phosgene, based on the total weight of the phosgene-comprising gas stream, preferably from 75 to 100% by weight of phosgene, based on the total weight of the phosgene-comprising gas stream, more preferably from 90 to 99.9% by weight of phosgene, based on the total weight of the phosgene-comprising gas stream, is heated to a temperature which is at least 5°C greater than the vaporization temperature of the amine under the operating conditions (pressure) in the fluidized-bed reactor (R) before being fed into the fluidized-bed reactor.

5. The process according to any of claims 1 to 4, wherein the amine-comprising liquid stream (2) comprising from 50 to 100% by weight of amine, based on the total weight of the amine-comprising liquid stream, preferably from 75 to 100% by weight of amine, based on the total weight of the amine-comprising liquid stream, more preferably from 90 to 99.99% by weight of amine, based on the total weight of the amine-comprising liquid stream, is preheated to a temperature which is at least 1°C below, preferably at least 5°C below, the boiling point of the amine under the operating conditions in the fluidized-bed reactor directly before being fed into the fluidized bed.

6. The process according to any of claims 1 to 5, wherein a mixture comprising the reaction gas mixture and the inert solid flows out of the fluidized-bed reactor (R) in the upper region of the reactor (R) and is passed to a separation.

7. The process according to claim 6, wherein the separation into the reaction gas mixture and the inert solid is carried out in one or more apparatuses selected from the group consisting of cyclones and filters.

8. The process according to any of claims 1 to 7, wherein from 50 to 100%, preferably from 75 to 100%, particularly preferably from 90 to 100%, of the amine groups used have been reacted in the reaction gas mixture which is separated off.

9. The process according to any of claims 1 to 8, wherein the reaction gas mixture is fed to a subsequent residence time reactor to achieve complete reaction of the amine groups.

10. The process according to any of claims 1 to 9, wherein the reaction gas mixture is cooled in one or more stages by direct or indirect heat transfer and a liquid phase is optionally condensed out during cooling.

11. The process according to any of claims 1 to 10, wherein the solid is partly or completely recirculated to the lower part of the fluidized bed.

12. The process according to any of claims 1 to 11, wherein the solid is worked up partly or completely, preferably in a second fluidized bed, and the worked up solid is partly or completely recycled to the fluidized-bed reactor (R), particularly preferably with the work-up of the solid being effected essentially by burning-off of carbon-comprising deposits on the solid.

13. The process according to any of claims 1 to 12, wherein the inert solid is a material which is inert toward all reactants and reaction products, preferably an inorganic material, particularly preferably SiO₂ or Al₂O₃, in particular α-Al₂O₃.

14. The process according to any of claims 1 to 13, wherein the average particle size of the inert solid is in the range from 20 µm to 2000 µm, preferably in the range from 20 µm to 500 µm and particularly preferably in the range from 50 µm to 150 µm.

15. The process according to any of claims 1 to 14, wherein the amine used is a diamine, preferably hexamethylenediamine, isomers and isomer mixtures of tolylenediamine or methylenedi(phenylamine), particularly preferably an isomer mixture of tolylenediamine.

16. The process according to any of claims 1 to 15, wherein the amine-comprising and/or phosgene-comprising feed stream is diluted with an inert component, preferably nitrogen.

17. The process according to any of claims 1 to 16, wherein the liquid stream comprising the amine is fed into the fluidized bed by means of one or more nozzles which are configured as single-fluid or two-fluid nozzles, preferably as two-fluid nozzles, with the atomization gas in the case of two-fluid nozzles preferably being a phosgene-comprising gas.

18. The process according to claim 17, wherein the two-fluid nozzles in the case of a phosgene-comprising gas as atomization gas preferably being configured as externally mixing.

## Revendications

1. Procédé pour la préparation d'isocyanates par phosgénation des amines correspondantes dans un réacteur à lit tourbillonnant (R), **caractérisé en ce que**
- on utilise un flux gazeux (1), contenant le phosgène, comme gaz tourbillonnant et on maintient un solide inerte en suspension et **en ce que**
- on dose un flux liquide (2), contenant l'amine, dans la couche tourbillonnante,
- l'amine étant partiellement ou complètement évaporée et amenée à réagir avec le phosgène avec obtention d'un mélange gazeux réactionnel, contenant l'isocyanate correspondant, qui est soutiré du réacteur à couche tourbillonnante (R).

2. Procédé selon la revendication 1, **caractérisé en ce que** le réacteur à couche tourbillonnante (R) est un appareil à symétrie de rotation présentant un axe longitudinal vertical.

3. Procédé selon la revendication 2, **caractérisé en ce que** le réacteur à couche tourbillonnante (R) est un appareil principalement cylindrique.

4. Procédé selon l'une quelconque des revendications 1 à 3, **caractérisé en ce que** le flux gazeux contenant du phosgène (1), contenant 50 à 100% en poids de phosgène, par rapport au poids total du flux gazeux contenant du phosgène, de préférence 75 à 100% en poids de phosgène, par rapport au poids total du flux gazeux contenant du phosgène, plus préférablement 90 à 99,9% en poids de phosgène, par rapport au poids total du flux gazeux contenant du phosgène, est chauffé, avant son introduction dans le réacteur à couche tourbillonnante, à une température qui est supérieure d'au moins 5°C à la température d'évaporation de l'amine dans les conditions de fonctionnement (pression) dans le réacteur à couche tourbillonnante (R).

5. Procédé selon l'une quelconque des revendications 1 à 4, **caractérisé en ce que** le flux liquide contenant de l'amine (2), contenant 50 à 100% d'amine, par rapport au poids total du flux liquide contenant de l'amine, de préférence 75 à 100% en poids d'amine, par rapport au poids total du flux liquide contenant de l'amine, plus préférablement 90 à 99,99% en poids d'amine, par rapport au poids total du flux liquide contenant de l'amine, est préchauffé, juste avant le dosage dans la couche tourbillonnante, à une température qui est inférieure d'au moins 1°C, de préférence d'au moins 5°C, à la température d'ébullition de l'amine dans les conditions de fonctionnement dans le réacteur à couche tourbillonnante.

6. Procédé selon l'une quelconque des revendications 1 à 5, **caractérisé en ce qu'**un mélange s'écoule du réacteur à couche tourbillonnante (R), de la partie supérieure de celui-ci, qui contient le mélange gazeux réactionnel ainsi que le solide inerte et qui est introduit dans une séparation.

7. Procédé selon la revendication 6, **caractérisé en ce que** la séparation, en mélange gazeux réactionnel d'une part et en solide inerte d'autre part, est réalisée dans un ou plusieurs appareils, choisis dans le groupe formé par un cyclone et un filtre.

8. Procédé selon l'une quelconque des revendications 1 à 7, **caractérisé en ce que**, dans le mélange gazeux réactionnel séparé, 50 à 100%, de préférence 75 à 100%, de manière particulièrement préférée 90 à 100% des groupes amine utilisés sont transformés.

9. Procédé selon l'une quelconque des revendications 1 à 8, **caractérisé en ce que** le mélange gazeux réactionnel est introduit dans un réacteur consécutif, à durée de séjour, pour la transformation complète des groupes amine.

10. Procédé selon l'une quelconque des revendications 1 à 9, **caractérisé en ce que** le mélange gazeux réactionnel est refroidi en une ou plusieurs étapes, par transfert thermique direct ou indirect et une phase liquide est le cas échéant séparée par condensation.

11. Procédé selon l'une quelconque des revendications 1 à 10, **caractérisé en ce que** le solide est recyclé partiellement ou complètement dans la partie inférieure de la couche tourbillonnante.

12. Procédé selon l'une quelconque des revendications 1 à 11, **caractérisé en ce que** le solide est traité partiellement ou complètement, de préférence dans une deuxième couche tourbillonnante, et **en ce que** le solide traité est recyclé partiellement ou complètement dans le réacteur à couche tourbillonnante (R), de manière particulièrement préférée **en ce que** le traitement du solide a lieu essentiellement par combustion de dépôts contenant du carbone sur le solide.

13. Procédé selon l'une quelconque des revendications 1 à 12, **caractérisé en ce qu'**il s'agit, pour le solide inerte, d'un matériau inerte par rapport à tous les partenaires et produits de réaction, de préférence d'un matériau inorganique, de manière particulièrement préférée de SiO₂ ou d'Al₂O₃, en particulier d'α-Al₂O₃.

14. Procédé selon l'une quelconque des revendications 1 à 13, **caractérisé en ce que** la grosseur moyenne des particules du solide inerte se situe dans la plage de 20 µm à 2000 µm, de préférence dans la plage de 20 µm à 500 µm, et de manière particulièrement préférée dans la plage de 50 µm à 150 µm.

15. Procédé selon l'une quelconque des revendications 1 à 14, **caractérisé en ce qu'**il s'agit, pour l'amine utilisée, d'une diamine, de préférence d'hexaméthylènediamine, d'isomères et de mélanges d'isomères de la toluylènediamine ou de la méthylènediphényldiamine, de manière particulièrement préférée d'un mélange d'isomères de la toluylènediamine.

16. Procédé selon l'une quelconque des revendications 1 à 15, **caractérisé en ce que** le flux de départ contenant de l'amine et/ou du phosgène est dilué par un composant inerte, de préférence de l'azote.

17. Procédé selon l'une quelconque des revendications 1 à 16, **caractérisé en ce que** le flux liquide, contenant de l'amine, est dosé dans la couche tourbillonnante au moyen d'un ou de plusieurs pulvérisateurs, qui sont conçus comme pulvérisateurs à une ou à deux substances, de préférence comme pulvérisateurs à deux substances, le gaz de vaporisation des pulvérisateurs à deux substances étant de préférence un gaz contenant du phosgène.

18. Procédé selon la revendication 17, **caractérisé en ce que** les pulvérisateurs à deux substances, dans le cas d'un gaz contenant du phosgène comme gaz de vaporisation, sont de préférence conçus comme pulvérisateurs à mélange externe.
